# FASCICULE DE BREVET EUROPEEN

(11) **EP 1 476 434 B1**
(45) Date de publication et mention de la délivrance du brevet: **14.06.2006**
(21) Numéro de dépôt: 03717380.4
(22) Date de dépôt: 07.02.2003
(51) Int. Cl.: C07D 237/04, A61K 31/50, A61P 35/00

(54) **DERIVES DE LA PYRIDAZINE, LEUR UTILISATION COMME MEDICAMENTS, ET LEUR PROCEDE DE PREPARATION**
PYRIDAZINDERIVATE, DEREN VERWENDUNG ALS HEILMITTEL, UND DEREN VERFAHREN ZU IHRER VERWENDUNG
PYRIDAZINE DERIVATIVES, USE OF THE SAME AS MEDICAMENTS, PHARMACEUTICAL COMPOSITIONS AND METHOD FOR PRODUCING THE SAME

(30) Priorité: 11.02.2002 FR 0201632
(43) Date de publication de la demande: 17.11.2004
(73) Titulaire: Aventis Pharma S.A., 92160 Antony (FR)
(72) Inventeur: BHATNAGAR, Neerja, F-91600 Savigny sur Orge (FR); BENARD, Didier, Attainville, 9557 0 Bouffemont (FR); BROTO, Pierre, F-75019 Paris (FR); GOURVEST, Jean-François, F-77410 Claye Souilly (FR); MAUGER, Jacques, Tucson, AZ 85704 (US)
(74) Mandataire: Rousseau, Pierrick Edouard
(86) Numéro de dépôt international: PCT/FR2003/000381
(87) Numéro de publication internationale: WO 2003/068140

(56) Documents cités:
- EP-A- 0 621 270

## Description

L'invention concerne de nouveaux dérivés de pyridazine, leur préparation, leur utilisation comme médicaments, notamment comme inhibiteurs de la cathépsine K., ainsi que les compositions pharmaceutiques les renfermant.

Les enzymes métaboliques telles que des protéases ou des kinases sont des enzymes largement distribuées dans le règne animal. A titre d'exemples non exhaustifs, on peut citer comme références bibliographiques pour les protéases, les documents : « Methods in Enzymology XLII (1975) » et « Journal of Médicinal Chemistry » vol. 43 n° 3 (D. Leung, G. Abbenante et D.P. Fairlie) et pour les kinases, le document : « Methods in Enzymology », Vol 80 (1981) (Academic Press Inc.).

Parmi les protéases capables de catalyser sélectivement l'hydrolyse des laisons polypeptidiques, on peut citer les quatre principales classes : protéase aspartique, sérine, cystéine et métallo-protéase.

Comme protéase aspartique on peut citer notamment la HIV-1 protéase, la rénine, les plasmepsines, la cathépsine D.

Comme sérine protéase on peut citer notamment la thrombine, le facteur Xa, l'élastase, la tryptase, les "complement de convertases", la protéase NS3 de l'hépatite C.

Parmi les cystéine-protéases, il existe trois groupes structurellement distincts, le groupe papaine et les cathepsines, le groupe ICE (les caspases) et le groupe picorna-viral (semblable aux sérine-protéases dans lesquelles la sérine est remplacée par une cystéïne).

Ainsi, on peut citer notamment la cathépsine K, la cathépsine B, la cathépsine L, la cathépsine S, les caspases, le rhinovirus 3C protéase et les papaines et calpaines.

Comme métalloprotéase, on peut citer notamment l'enzyme de conversion de l'Angiotensine, l'Endopeptidase neutre et le mélange des deux, la matrix metalloprotéase ainsi que la Tumor-necrosis Factor-α-Converting Enzyme.

Ces enzymes kinases ou protéases sont impliquées dans des processus de catabolisation et de communication inter et intracellulaire : elles jouent un rôle important dans un grand nombre de maladies de domaines différents tels que notamment le domaine cardiovasculaire, l'oncologie, le système nerveux central, l'inflammation, l'ostéoporose et également les maladies infectieuses, parasitaires, fongiques ou virales. C'est pourquoi ces protéines sont des cibles de grand intérêt pour la recherche pharmaceutique.

La demande de brevet EP 0 621 270 décrit des dérivés de la pyridazine inhibiteurs de la collagenase de type IV, utiles notamment contre les métastases cancéreuses.

La présente invention a ainsi pour objet les produits de formule (I) : 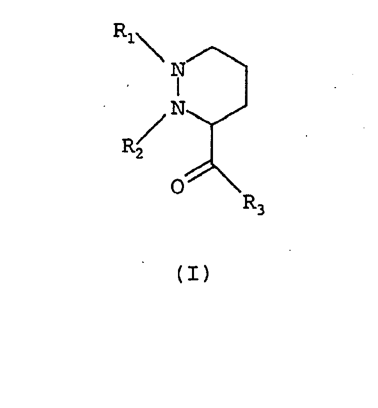 dans laquelle :
R₁ représente un atome d'hydrogène ou un groupement alkyle linéaire ou ramifié ayant de 1 à 6 atomes de carbone, COR, COOR, R étant choisi dans le groupe constitué par un radical alkyle linéaire ou ramifié renfermant de 1 à 6 atomes de carbone, éventuellement substitué par un radical pyridyle ou carbamoyle, un radical -CH₂-alkényle linéaire ou ramifié renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle linéaire ou ramifié renfermant de 1 à 6 atomes de carbone, alkoxy linéaire ou ramifié renfermant de 1 à 6 atomes de carbone ou par 1 à 3 atomes d'halogène,
R₂ représente un groupement répondant à la formule (II) suivante : 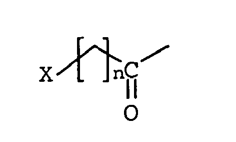 dans laquelle :
   n vaut 0,1,2 ou 3 ; une double liaison pouvant éventuellement être présente lorsque n vaut 2 ou 3;
   X est l'un des groupements:
      groupement hétérocyle monocyclique ou bicyclique saturé ou insaturé; ou
      un groupement aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle linéaire ou ramifié renfermant de 1 à 6 atomes de carbone, alkoxy linéaire ou ramifié renfermant de 1 à 6 atomes de carbone ou par 1 à 3 atomes d'halogène ; ou
      un groupement NR₄R₅, R₄ étant un groupement alkyle linéaire ou ramifié ayant de 1 à 6 atomes de carbone ou un groupement COR, CONHR, CSNHR ou SO₂R, R ayant la signification donnée précédemment et R₅ étant un atome d'hydrogène ou un radical alkyle linéaire ou ramifié ayant de 1 à 6 atomes de carbone ; ou
      un groupement COR, R ayant la signification donnée précédemment et R₅ étant un atome d'hydrogène ou un radical alkyle linéaire ou ramifié ayant de 1 à 6 atomes de carbone ; ou
R₃ est un groupement de formule -Y-(CH₂)ₘ-C(CN)R₆R₇ , dans laquelle :
   Y est un atome d'oxygène ou un groupement -N(R₈)-,
   R₈ étant un atome d'hydrogène ou un groupement alkyle linéaire ou ramifié ayant de 1 à 6 atomes de carbone,
   m vaut 0,1,2 ou 3,
   R₆ est un atome d'hydrogène, un groupement alkyle linéaire ou ramifié ayant de 1 à 6 atomes de carbone ou un groupement aryle ou alkaryle, le noyau du radical aryle ou aralkyle étant éventuellement substitué par un radical OH, NH₂, NO₂, alkyle linéaire ou ramifié renfermant de 1 à 6 atomes de carbone, alkoxy linéaire ou ramifié renfermant de 1 à 6 atomes de carbone, aryloxy renfermant de 7 à 11 atomes de carbone, ce groupement aryloxy étant lui-même éventuellement substitué par 1 à 3 halogènes,
   R₇ est un atome d'hydrogène ou un groupement alkyle linéaire ou ramifié ayant de 1 à 6 atomes de carbone,
   ou R₆ et R₇ pouvant former ensemble un cycle saturé à 6 chaînons ;
   lesdits produits de formule (I) étant sous toutes les formes isomères possibles, racémiques, énantiomères et diastéréo-isomères ;
   ainsi que les sels d'addition avec les acides minéraux et organiques ou avec les bases minérales et organiques de ces produits.

Les produits de la présente invention tels que définis ci-dessus et ci-après possèdent des propriétés inhibitrices d'enzymes métaboliques telles que définies ci-dessus notamment de kinases ou de protéases comme notamment les cystéine protéases ou sérine protéases.

Les produits de la présente invention peuvent ainsi notamment être utiles dans la prévention ou le traitement de maladies dans lesquelles de telles enzymes métaboliques sont impliquées comme certaines maladies cardiovasculaires, maladies du système nerveux central, maladies inflammatoires, maladies de l'os telles que par exemple l'ostéoporose, maladies infectieuses nécessitant notamment pour leur thérapie des anti-infectieux ou encore certains cancers.

Dans les produits de formule (I) et dans ce qui suit :
- le terme aryle renfermant de 6 à 10 atomes de carbone désigne un radical insaturé, comportant un ou deux cycles fusionnés, éventuellement interrompu par un à trois hétéroatomes choisis parmi azote, oxygène et soufre. On peut citer: phényle, naphtyle.
- le terme aralkyle renfermant de 7 à 11 atomes de carbone désigne un radical aryle tel que ci-dessus, lié par un radical alkyle linéaire ou ramifié, ce radical alkyl ayant de 1 à 5 atomes de carbone. On peut citer notamment le benzyle.
- le terme aralkyloxy indique la présence d'un oxygène terminal sur le groupe aralkyle précité.
- le terme radical hétérocyclique monocyclique désigne un radical saturé ou insaturé constitué de 5 ou 6 chaînons tel que l'un ou plusieurs des chaînons représente un atome d'oxygène, de soufre ou d'azote : un tel radical hétérocyclique désigne ainsi un radical carbocyclique interrompu par un ou plusieurs hétéroatomes choisis parmi les atomes d'oxygène, d'azote ou de soufre étant entendu que les radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents. On peut citer notamment le radical dioxolane, dioxane, dithiolane, thiooxolane, thiooxane, morpholinyle, pipérazinyle, pipérazinyle substitué par un radical alkyle, linéaire ou ramifié, renfermant au plus 4 atomes de carbone, pipéridyle, thiényle tel que 2-thiényle et 3-thiényle, furyle tel que 2 furyle, pyrimidinyle, pyridyle tel que 2-pyridyle, 3-pyridyle et 4-pyridyle pyrimidyle, pyrrolyle, thiazolyle, isothiazolyle, diazolyle, thiadiazolyle, triazolyle, tétrazolyle libre ou salifié thiadiazolyle, thiatriazolyle, oxazolyle, oxadiazolyle, 3- ou 4-isoxazolyle. On peut citer tout particulièrement les radicaux morpholinyle, thiényle tel que 2-thiényle et 3-thiényle, furyle tel que 2-furyle , tétrahydrofuryle, thiényle, tétrahydrothienyle, pyrrolyle, pyrrolinyle, pyridyle et pyrrolidinyle.
- le terme radical hétérocyclique bicyclique désigne un radical saturé ou insaturé constitué de 8 à 12 chaînons tel que l'un ou plusieurs des chaînons représente un atome d'oxygène, de soufre ou d'azote et notamment des groupes hétérocycliques condensés contenant au moins un hétéroatome choisi parmi le soufre, l'azote et l'oxygène, par exemple benzothiényle tel que 3-benzothiényle, benzothiazolyle, quinolyle, tetralone, benzofuryle, benzopyrrolyle, benzimidazolyle, benzoxazolyle, thionaphtyle, indolyle ou purinyle.

Les composés de formule (1) peuvent être salifiés par les groupements divers connus de l'homme du métier parmi lesquels on peut citer, par exemple :
- parmi les composés de salification, des bases minérales telles que, par exemple, un équivalent de sodium, de potas-sium, de lithium, de calcium, de magnésium ou d'ammonium ou des bases organiques telles que, par exemple, la méthylamine, la propylamine, la triméthylamine, la diéthylamine, la tri- éthylamine, la N,N-diméthyléthanolamine, le tris (hydroxy-méthyl)amino méthane, l'éthanolamine, la pyridine, la pico-line, la dicyclohexylamine, la morpholine, la benzylamine, la procaïne, la lysine, l'arginine, l'histidine, la N-méthyl- glucamine,
- Les sels d'addition avec les acides minéraux ou organiques des produits de formule (1) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a notamment pour objet les produits de formule (I) telle que définie ci-dessus dans laquelle R₁ est un atome d'hydrogène, un groupement méthyle, benzyle, -COO-benzyle ou -CO-méthylène-benzyle, en particulier, ceux dans lesquels n est égal à 0 ou 2.

De préférence, R₅ est un atome d'hydrogène.

Sont également intéressants les produits de formule (I) dans lesquels que X est un groupement phényle, -NHCO-benzyle ou ceux dans lesquels X est le groupement

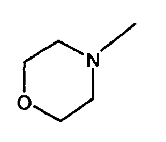

De préférence, R₆, R₇ et/ou R₈ sont, indépendamment les uns des autres, un atome d'hydrogène.

R₆ peut avantageusement être le le groupement phényle, -C₆H₄-O-C₆H₅ ou -C₂H₄-O-C₆H₄Br.

Présentent également un intérêt, les produits de formule (I) dans lesquels m vaut 0 ou 2.

La présente invention a également tout particulièrement pour objet certains produits de formule (I).

### Procédé selon l'invention

La présente invention a ainsi également pour objet un procédé de préparation des produits de formule (I), telle que définie ci-dessus, caractérisé en ce qu'il comporte l'étape de réaction d'un produit de formule (IV) 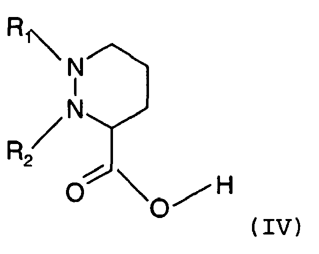 dans laquelle R₁ et R₂ ont la même signification que ci-dessus,
avec un amino-nitrile ou cyano-hydrine de formule HR₃, dans laquelle R₃ a la même signification que ci-dessus, pour obtenir un produit de formule (I).

La présente invention a ainsi également pour objet un procédé de préparation des produits de formule (I), telle que définie ci-dessus, caractérisé en ce qu'il comporte :
1) une étape au cours de laquelle on fait réagir un produit de formule (II) 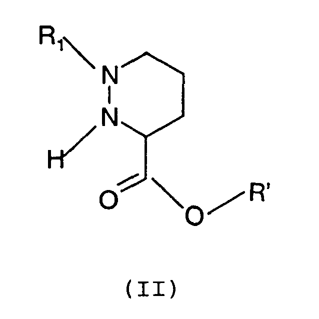 avec un chlorure d'acide de formule R₂Cl, dans laquelle R₁ et R₂ ont les mêmes significations que ci-dessus et R' est un groupement alkyle linéaire ou ramifié ayant de 1 à 6 atomes de carbone, pour obtenir le produit de formule (III); 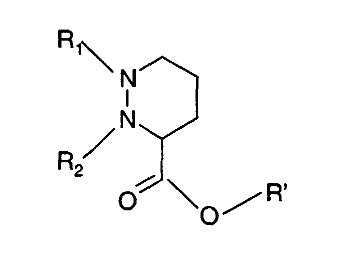
2) une étape au cours de laquelle on hydrolyse le produit de formule (III) obtenu à l'étape 1) en le produit de formule (IV); 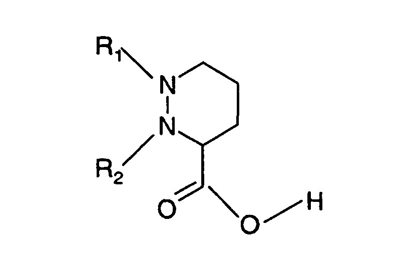
3) une étape au cours de laquelle on fait réagir le produit de formule (IV) obtenu à l'étape 2) avec un amino-nitrile ou cyano-hydrine de formule HR₃, dans laquelle R₃ a la même signification que ci-dessus, pour obtenir un produit de formule (I).

Le premier procédé décrit a comme produit de départ un acide pyridazine-carboxylique, de formule (IV), que l'on fait réagir directement avec un dérivé nitrile approprié.

Le second procédé est basé sur la préparation d'un ester intermédiaire.

Un tel procédé est donc constitué essentiellement des 3 étapes suivantes :
- l'étape 1) permet d'obtenir le produit de formule (III) 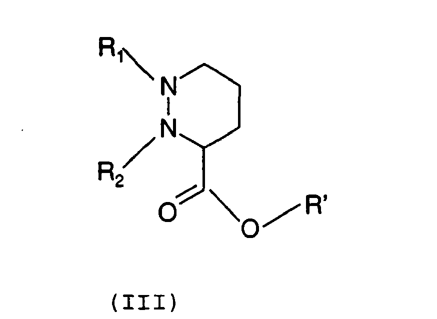 à partir du produit de formule (II) ;
- l'étape 2), qui est un étape connue en soi d'hydrolyse d'un ester et a lieu généralement en présence d'une base, permet d'obtenir le produit de formule (IV) 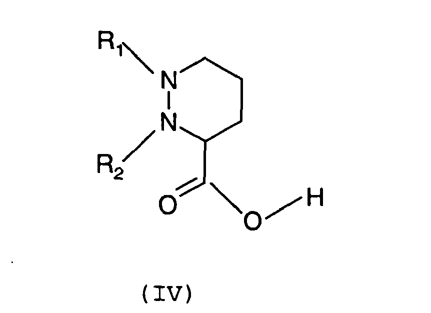 à partir du produit de départ de formule (III) ;
- l'étape 3) permet d'obtenir le produit de formule (I) à partir du produit de formule (IV).
   Quant aux étapes optionnelles, elles sont d'une manière générale des réactions classiques, bien connues de l'homme du métier.
   Ainsi, les fonctions réactives qu'il convient, le cas échéant, de protéger sont généralement les fonctions acides carboxyliques, amines, amides et hydroxy.
   La protection de la fonction acide est notamment effectuée sous forme d'esters d'alkyle, d'esters allyliques; de benzyle, benzhydryle ou p-nitrobenzyle.
   La déprotection est effectuée par saponification, hydrolyse acide, hydrogénolyse, ou encore clivage à l'aide de complexes solubles du Palladium O.
   La protection des amines et amides est notamment effectuée sous forme de dérivés benzylés, sous forme de carbamates, notamment d'allyle, benzyle, phényle ou tertbutyle, ou encore sous forme de dérivés silylés tels que les dérivés tertbutyle diméthyl, triméthyl, triphényl
ou encore diphényl tertbutyl-silyle.

La déprotection est effectuée, selon la nature du groupement protecteur, par le sodium ou le lithium dans l'ammoniac liquide, par hydrogénolyse ou à l'aide de complexes solubles du Palladium O, par action d'un acide, ou par action du fluorure de tétrabutylammonium.

La protection des alcools est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

La réaction d'amidification est effectuée au départ de l'acide carboxylique à l'aide d'un agent d'activation tel qu'un chloroformiate d'alkyle ou l'EDCI, par action de l'ammoniaque ou d'une amine appropriée ou de leurs sels d'acides.

Les réactions d'acylation et de sulfonylation sont effectuées sur les hydroxyurées par action respectivement d'un halogènure ou anhydride d'acide carboxylique approprié ou d'un halogénure d'acide sulfonique approprié.

La réaction d'alkylation est effectuée par action sur les dérivés hydroxylés d'un halogènure d'alkyle ou d'alkyle substitué, notamment par un radical carboxy libre ou estérifié.

L'introduction finale éventuelle d'une double liaison, est effectuée par action d'un dérivé halogéné du sélénium puis oxydation, selon des méthodes connues de l'homme du métier.

La formation d'un groupement urée est effectuée de préférence par action d'un isocyanate approprié sur le NH libre.

La réduction d'acides en alcools peut être effectuée par action d'un borane ou via un anhydride mixte intermédiaire, par action d'un borohydrure alcalin. L'anhydride mixte est préparé par exemple à l'aide d'un chloroformiate d'alkyle.

La déshydratation d'amide en nitrile peut intervenir dans les conditions des réactions de carbonylation et cyclisation.

La salification par les acides est le cas échéant réalisée par addition d'un acide en phase soluble au composé. La salification par les bases peut concerner soit les composés comportant une fonction acide, notamment carboxy, soit ceux comportant une fonction sulfooxy ou ceux comportant un hétérocycle à caractère acide. Dans le premier cas, on opère par addition d'une base appropriée telle que celles citées précédemment. Dans le second cas, on obtient directement le sel de pyridinium lors de l'action du complexe SO₃-pyridine et on obtient les autres sels à partir de ce sel de pyridinium. Dans l'un ou l'autre cas, on peut encore opérer par échange d'ions sur résine. Des exemples de salifications figurent ci-après dans la partie expérimentale.

La séparation des énantiomères et diastéréoisomères peut être réalisée selon les techniques connues de l'homme du métier, notamment la chromatographie.

La dernière étape du procédé selon l'invention peut, la cas échéant, être suivie d'une hydrogénolyse de manière à convertir le groupement R₁ en un atome d'hydrogène.

Des illustrations de telles réactions définies ci-dessus sont données dans la préparation des exemples décrits ci-après.

Les produits de formule (I) tels que définis ci-dessus ainsi que leurs sels d'addition avec les acides présentent d'intéressantes propriétés pharmacologiques.

Les produits de la présente invention peuvent ainsi être doués de propriétés inhibitrices d'une ou plusieurs enzymes métaboliques telles que définies ci-dessus notamment de kinases ou de protéases.

Certains produits de formule (I) de la présente invention tels que définis ci-dessus, peuvent donc notamment posséder des propriétés inhibitrices de certaines protéines kinases ou de protéases.

A titre de protéines kinases d'intérêt, on peut viser les cathepsines B, H, J, L, N, S, T, C, V W, K ou O, O2; notamment celles impliquées dans les maladies du métabolisme du cartilage et de l'os et les cancers des os, et tout particulièrement la cathepsine K.

Les niveaux, la régulation et l'activité d'un certain nombre de protéines kinases ou protéases jouent un rôle dans plusieurs pathologies humaines. L'activité d'une protéine kinase ou protéase peut notamment être associée à des récepteurs possédant des domaines transmembranaires ou à des protéines intracellulaires.

Certaines kinases ou protéases peuvent jouer un rôle dans l'initiation, le développement et l'achèvement des évènements du cycle cellulaire et ainsi, des molécules inhibitrices de telles kinases ou protéases sont susceptibles de limiter des proliférations cellulaires non désirées telles que celles observées dans les cancers, psoriasis, croissance de champignons, de parasites (animaux, protistes): de telles molécules inhibitrices de ces kinases ou protéases sont ainsi également susceptibles d'intervenir dans la régulation de maladies neurodégénératives telles que la maladie d'Alzheimer.

Certains produits de formule (I) de la présente invention peuvent ainsi être doués de propriétés antimitotiques.

Certains produits de formule (I) telle que définie ci-dessus peuvent comme inhibiteurs de kinase ou protéase avoir notamment la propriété d'inhiber la résorption osseuse médiée par les ostéoclastes. Ils peuvent donc être utiles pour le traitement thérapeutique ou prophylactique de maladies qui sont causées au moins en partie par une augmentation non désirée de la résorption osseuse, par exemple l'ostéoporose.

Certains produits de formule (I) de la présente invention peuvent ainsi par exemple inhiber l'adhésion des ostéoclastes sur la surface de l'os et ainsi la résorption osseuse par les ostéoclastes.

Les maladies de l'os dont le traitement ou la prévention nécessitent l'emploi des composés de formule (I), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, la maladie de Paget, l'ostéopénie induite par l'immobilisation. En outre les composés de formule (I) peuvent être utilisés pour soulager, empêcher ou traiter les désordres de l'os qui sont causés par les traitements, par les glucocorticoides, les thérapies liées à la prise de stéroides ou de corticostéroïdes ou par les déficiences d'hormones sexuelles mâles ou femelles.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

Certains produits de formule (I) de la présente invention peuvent posséder en plus de leurs propriétés inhibitrices spécifiques de kinases ou protéases, des effets cellulaires intéressants tels que des propriétés antiprolifératives et notamment des effets sur l'apoptose.

On sait par des travaux décrits dans la littérature tel que dans WO 97/20842, que des rapports existent entre le cycle cellulaire et l'apoptose. Parmi les voies conduisant à l'apoptose, certaines sont dépendantes de kinases ou de protéases.

Les produits de la présente invention sont notamment utiles pour la thérapie de tumeurs.

Les produits de l'invention peuvent également ainsi augmenter les effets thérapeutiques d'agents anti-tumoraux couramment utilisés.

Les produits de formule (I) de la présente invention possèdent aussi des propriétés antimitotiques et anti-neurodégénératives.

Certains produits de la présente invention peuvent être inhibiteurs d'effets vasoconstricteurs et hypertenseurs et ainsi produire un effet anti-ischémique, ou encore s'opposer à des effets stimulants au niveau de certains types cellulaires notamment les cellules musculaires lisses, les fibroblastes, les cellules neuronales et les cellules osseuses.

Les produits selon la présente invention peuvent ainsi être utilisés dans le traitement de maladies telles que les maladies prolifératives, le cancer, la resténose, l'inflammation; les allergies, les maladies cardiovasculaires ou certaines maladies infectieuses.

Les produits de la présente invention peuvent également être utilisés dans le traitement de certains désordres gastro-intestinaux, gynécologiques et en particulier pour un effet relaxant au niveau de l'utérus.

Les produits de formule (I) de la présente demande peuvent ainsi posséder d'intéressantes propriétés pharmacologiques justifiant leur application en thérapeutique.

L'invention a donc aussi pour objet les composés selon l'invention pour leur utilisation à titre de médicaments, destinés à la prévention ou au traitement de maladies rappelées ci-dessus.

L'invention a tout particulièrement pour objet les compositions pharmaceutiques contenant à titre de principe actif l'un au moins des composés selon l'invention en association avec un support pharmaceutiquement acceptable.

Les compositions pharmaceutiques de la présente invention telles que définies ci-dessus peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

L'invention a encore pour objet l'utilisation des composés selon l'invention pour la fabrication de médicaments, destinés à la prévention ou au traitement de maladies rappelées ci-dessus.

Les produits des exemples suivants sont caractérisés par leurs spectres RMN (300Hz dans du CDCl₃ ou du DMSO) et par leur mass moléculaire MM (électrospray en mode positif; résultats sous forme de la masse de l'ion moléculaire H+ ou sous forme d'adduit du sodium).

Les produits de départ de formule (II) sont connus ou peuvent être préparés selon des méthodes connues de l'homme du métier. Des références de littérature ainsi que des préparations sont éventuellement fournies ci-après dans la partie expérimentale.

Les exemples suivants illustrent l'invention, sans toutefois en limiter la portée.

### Exemples

Dans les exemples qui suivent les abréviations suivantes ont été utilisées:
M: masse molaire moléculaire
SM: spectrométrie de masse
EDCI:1-(3-diméthylamino-propyl)-3-éthylcarbo-diimide chlorhydrate
AcOEt : acétate d'éthyle
DMF : N,N-diméthylformamide
HOBt : 1-hydroxybenzotriazole hydrate
DMSO : diméthylsulfoxyde

### Exemple 1

### Synthèse d'un acide R₂OH

10,69 g (0,12 mM) de β-alanine sont mis en solution dans 60 ml de NaOH 2N (0,12mM). On ajoute goutte à goutte 17,6 µl (0,132mM) du chlorure d'acide benzyl-méthanoïque et 66 ml (0,132mM) de NaOH 2N.

On garde le mélange sous agitation pendant 1h30 à 0°C. Le milieu réactionnel est extrait 2 fois par 25 ml de diethyl ether puis amené à pH=3 par 65 ml d'une solution HCl 2N. On filtre le précipité formé, le sèche. On récupère 23,4 g d'un solide blanc correspondant à l'acide 3-[(phénylacétyl)amino]propanoïque.

Le rendement correspondant est de 94,2 %.

### Spectre RMN du proton

Dans le DMSO, à 300 MHz, déplacements chimiques des pics en ppm et multiplicité:
2, 5 (t, 2Hₐ) ; 3, 35 (q, 2H_{b}) ; 3, 5 (s, 1H_{c}) ; 7,3 à 7,5 (m, 5H) ; 8, 25 (t, 1H)

### Exemple 2

### Synthèse d'un acide intermédiaire

### Etape A

291 mg (1,4 mM) de l'acide obtenu à l'exemple 1 sont mis en solution dans 5 ml de dichlorométhane et 0,5 ml de DMF, on ajoute goutte à goutte 368 ml (4,21 mM) de chlorure d'oxalyle.

Après 3 h d'agitation à température ambiante, le chlorure d'acide est formé et utilisé tel quel pour la suite de la synthèse.

On ajoute à la solution précédente, 390 mg (1,4 mM) de dérivé pyridazine en solution dans 5 ml de dichlorométhane et 733 ml de DMF, ledit dérivé de pyridazine correspondant au (3S) tétrahydro-1,3(2H)-pyridazinedicarboxylate de 1-(phénylméthyle)-3-méthyle. Ce composé est obtenu par estérification de l'acide pyridazine-3-carboxylique correspondant (voir aussi la description en tant que produit intermédiaire dans les documents WO-A-9955724, WO-A-9722619 et EP-A-25941).

On garde le milieu réactionnel sous agitation à température ambiante pendant 12 heures.

Celui-ci est concentré et purifié par chromatographie flash avec un mélange acétate d'ethyle/dichlorométhane : 80/20. On récupère 150 mg du produit attendu, correspondant au (3S) 2-[1-oxo-3-[(phénylacétyl)amino] propyl]-hexahydro-1,3(2H)-pyridazinedicarboxylate de 1-(phénylméthyle)-3-méthyle.

Le rendement correspondant est de 26%.

### Spectre RMN du proton

Dans le CDCl₃, à 300 MHz, déplacements chimiques des pics en ppm et multiplicité
1,46 et 2,04 (m, 2Hₑ) ; 1,72 et 2,1 (m, 2H_{d}), 2,61 (m, 2H_{b}) ; 2,90 m , 4,04 et 4,14 (dl, 2H_{f}) , 3,47 (m, 2Hₐ) ; 3,48 (m, 2Hₕ) ; 3,54 (sl, 3H) ; 4,73 à 4,98 (m, 1H_{g}) ; 5, 18 (sl, 1H_{c}) ; 5,22 (sl, 1H_{g}) , 5,96 et 6, 07 (d, 1H) ; 7 à 7,4 (m, 10H).

### Etape B

38 mg (0,08mM) de l'ester obtenu à l'étape A sont mis en solution dans 2 ml de methanol. On ajoute 8,3 µl (0,16 mM) d'une solution de soude 2N. on laisse en contact pendant 4 heures à température ambiante. Le milieu réactionnel est concentré, extrait par 25 ml d'acétate d'ethyle puis acidifié par une solution HCl 1N jusqu'à pH=1 puis extrait par 25 ml d'une solution d'acétate d'éthyle. La solution organique est séchée, concentrée pour donner 31 mg d'une huile correspondant au dérivé acide carboxylique recherché.
Le rendement correspondant est de 84%.

### Spectre RMN du proton

Dans le CDCl₃, à 300 MHz, déplacements chimiques des pics en ppm et multiplicité :
1,46 et 1,75 (m, 2Hₑ) ; 1,67 et 2,31 (m, 2H_{d}), 2,47 (m, 2H_{b}) ; 2,90 m , 4,04 et 4,14 (dl, 2H_{f}), 3,47 (m, 2Hₐ) ; 3,48 (m, 2Hₕ) ; 4,73 à 4,98 (m, 1H_{g}) ; 5,18(sl, 1H_{c}) ; 5,22 (sl, 1H_{g}) , 5,96 et 6, 07 (d, 1H) ; 7 à 7,4 (m, 10H).

### Exemple 3

### Synthèse d'un amino-nitrile HR₃

9,59 g (29,8 mM) d'une solution à 70% dans l'éther de 3-phénoxybenzaldehyde cyanohydrine sont mis en solution dans 60 ml d'ethanol dans une bombe metallique d'une contenance de 100 ml en présence de 10 g de MgSO₄.

On y fait buller de l'ammoniaque gazeux pendant 1 h en maintenant le milieu à -10°C et l'on conserve sous agitation ce mélange à température ambiante pendant 18 h. Le mélange est ensuite filtré et évaporé à sec.
Le résidu est repris dans 50 ml d'une solution aqueuse pH=1, extrait par 50 ml d'acétate d'éthyle. La phase aqueuse est ensuite neutralisée par Na₂CO₃ puis extrait 2 fois par 50 ml d'acétate d'éthyle. A cette solution on ajoute 100 ml d'une solution HCl/AcOEt à 20%. La solution finale est concentrée pour donner 3,12 g d'une poudre beige correspondant au 3-phénoxy α-amino-benzènacétonitrile.
Le rendement correspondant est de 40%.

### Spectre RMN du proton

Dans le CDCl₃, à 300 MHz, déplacements chimiques des pics en ppm et multiplicité :
4,88 (sl, 1H) ; 7,0 (s, 1H_{d}) ; 7,02 (dd, 2H_{g}); 7.14 (tl, 1Hₑ) ; 7,19 (1H, Hₐ) ; 7,26 (dl, 1H_{b}) ; 7,36 (m, 2H_{f}) ; 7,37 (m, 1H).

### Exemple 4

31 mg de l'acide obtenu à l'exemple 2 sont mis en solution dans 1 ml de DMF. On ajoute au milieu porté à 0°C (bain glace-sel), 14 mg (0,1 mM) de HOBT (1-hydroxybenzotriazole) puis 20 mg (0,1mM) d'EDCI. Le mélange ramené à température ambiante est conservé 1 heure sous agitation. On ajoute ensuite au milieu réactionnel 15,3 mg (0.068 mM) de l'amine obtenue à l'exemple 3 en solution dans 2 ml de DMF et 36 µl (0,2 mM) de DIPEA (diisopropyléthylamine). On conserve le mélange 12 h à température ambiante puis on verse dans 25 ml d'eau. La solution est extraite par 25 ml d'AcOEt, séchée et concentrée. Le résidu obtenu est purifié par chromatographie flash. On récupère 22 mg du produit attendu, correspondant au (3S) 2-[1-oxo-3-[(phénylacétyl)amino]propyl]-3-[[[cyano(3-phénoxyphényl) méthyl]amino]carbonyl]-tétrahydro-1(2H)-pyridazinecarboxylate de (phénylméthyle).
Le rendement correspondant est de 50%.

### Spectre RMN du proton

Dans le CDCl₃, à 300 MHz, déplacements chimiques des pics en ppm et multiplicité :
1,46 à 1,75 (m, 2Hₑ) ; 1,67 et 2,31 (m, 2H_{d}) ; 2,47 (m, 2H_{b}) ; 2,9 (m, 1H_{f}) ; 3,47 (m,2Hₐ) ; 3,48 (m, 2Hₕ) ; 4,04 et 4,14 (dl, 1H_{f}) ; 4,73, 4,98 et 5,22 (m, 2H_{g}) ; 5,18 (sl, 1H_{c}) ; 6,07 (m, 1H) ; 5,96 et 6,03 (d, 1Hᵢ) ; 6,07 (m, 1H) ; 6,9 à 7,47 (m, 19H_{Ar}) ; 8.42 et 8.5 (dl, 1H) .

### Exemple 5

On met en solution 19 mg (0,029 mM) du mélange 50/50 obtenu à l'exemple 4 dans 2 ml d'éthanol. On ajoute à cette solution 10 mg de Pd/C à 10%. On conserve 15 h sous agitation à température ambiante. Le milieu réactionnel est filtré puis concentré. Le résidu obtenu est purifié par chromatographie flash pour donner 10 mg du produit attendu qui est un mélange 50/50 de 2 diastéréoisomères (S,S) et (S,R), correspondant au (3S) 2-[3-[(phénylacétyl)amino]-1-oxopropyl]-N-[cyano(3-phénoxyphényl)méthyl]-hexahydro-3-Pyridazinecarboxamide. Le rendement correspondant est de 67%.

### Spectre RMN du proton

Dans le CDCl₃, à 300 MHz, déplacements chimiques des pics en ppm et multiplicité :
(mélange des 2 diastéréoisomères 50/50): 1,58 (m, 2Hₑ); 1,70 et 2,37 (m, 2H_{d}) ; 2,33 et 2,94 (m, 2H_{b}) ; 2,68, 2,72, 2,91 et 3,01 (m, 2H_{f}) ; 3,27 et 3,37 (m, 1Hₕ) ; 3,52 (sl, 1Hₕ) ; 3,83 (sl), 3,96 (sl) et 3,16 (dl, 2Hₐ); 5,14 (sl, 1H_{c}) ; 5,89 et 5,97 (sl, 1H) ; 6,09 et 6,18 (d, 1Hᵢ) ; 8,24 et 8,49 (dl, 1H).

### Exemple 6

A partir du mélange obtenu à l'exemple 4, on sépare les deux diastéréoisomères purs que l'on hydrogène ensuite séparément, dans les mêmes conditions que pour l'exemple 5.
On obtient ainsi deux diastéréoisomères séparés purs (dont la formule est bien entendu la même que celle donnée à l'exemple 5).

### Exemple 7

On procède comme indiqué à l'exemple 4, sauf qu'au lieu d'utiliser l'amine obtenue à l'exemple 3, on utilise directement la 3-phénoxybenzaldehyde cyanohydrine (produit de départ ayant servi à préparer cette amine). On obtient ainsi un mélange de diastéréoisomères, correspondant au (3S) 2-[1-oxo-3-[(phénylacétyl)amino]propyl]-tétrahydro-1,3(2H)-Pyridazinedicarboxylate de 1-(phénylméthyle) 3-[(R)cyano(3-phénoxyphényl)méthyle].

### Spectre RMN du proton

Dans le DMSO, à 300 MHz, déplacements chimiques des pics en ppm et multiplicité :
1,49 et 1,79 (m, 2H) ; 1,80 et 1,90 (m, 2H) ; 2,30 et 2,54 (m, 2H) ; 3,01 et 4,09 (m, 2H) ; 3,36 (sl, 2H) ; 3,27 (m, 2H) ; 4,89 et 5,11 (m, 1H) ; 4,98 et 5,15 (m, 1H) ; 5,31 (sl, 1H) ; 6,49 et 6,59 (s1, 1H) ; 6,90 à 7,50 (m, 19H).

### Exemple 8

On déprotège le composé obtenu à l'exemple 7 par hydrogénation comme indiqué à l'exemple 5. On obtient ainsi un mélange de deux diastéréoisomères correspondant au (3S) 2-[1-oxo-3-[(phénylacétyl)amino]propyl]-tétrahydro-1,3(2H)-Pyridazinecarboxylate de 3-[cyano(3-phénoxyphényl)méthyle].

### Exemple 9

On procède comme indiqué à l'exemple 4, sauf qu'au lieu d'utiliser l'amine obtenue à l'exemple 3, on utilise de la 3-(p-bromophénoxy)benzaldéhyde cyanohydrine du commerce .
On obtient ainsi un mélange de 2 diastéréoisomères 50/50 correspondant au (3S) 2-[1-oxo-3-[(phénylacétyl) amino]propyl]-tétrahydro-1,3(2H)-Pyridazinedicarboxylate de 1-(phénylméthyle) 3-[cyano-3-[(4-bromophénoxy) phényl]méthyle].

### Spectre RMN du proton

Dans le DMSO, à 300 MHz, déplacements chimiques des pics en ppm et multiplicité :
1,51 et 1,78 (m, 2Hₑ) ; 1,76 et 1,97 (m, 2H_{d}) ; 2,24 et 2,54 (m, 2H_{b}) ; 3,22 (m, 2H_{b}) ; 3,35 (m, 2Hₕ) ; 4,03 (m, 2H_{f}) ; 5,12 (sl, 1H_{g}); 5,18 (sl, 1H_{g}) ; 5,36 (m, 1H_{c}); 6,61 (m, 1Hᵢ) ; 7,01 à 7,57 (m, 18H_{Ar}) .
SM (Electrospray négatif) m/z : [M]⁻ = 737

### Exemple 10

On procède comme indiqué à l'exemple 4, sauf qu' au lieu d'utiliser l'amine obtenue à l'exemple 3, on utilise du (cyano amino méthyl) benzène du commerce.
On obtient ainsi un composé correspondant au (3S) 3-[[(cyanophénylméthyl)amino]carbonyl]-2-[1-oxo-3-[(phényl acétyl)amino]propyl]-tétrahydro-1(2H)-Pyridazine carboxylate de (phénylméthyle).
SM (Electrospray positif) m/z : [MH]⁺=568,3 [MNa]⁺= 590,2

### Exemple 11

On déprotège le composé obtenu à l'exemple 10, par hydrogénation comme indiqué à l'exemple 5.
On obtient le composé correspondant au (3S) N-(cyanophénylméthyl)-2-[1-oxo-3-[(phénylacétyl)amino] propyl]-hexahydro-3-Pyridazinecarboxamide.

### Spectre RMN du proton

Dans le CDCl₃, à 300 MHz, déplacements chimiques des pics en ppm et multiplicité :
1,58 (m, 2H) ; 1,71 (m,1H) ; 2,35 (m,1H) ; 2,28 et 2,98 (m, 1H) ; 2,38 et 2,87 (m,1H) ; 2,70 et 2,97 (m, 2H) ; 3,16 et 3,92 (m, 1H) ; 3,19 et 3,77 (m, 1H) ; 3,54 (sl, 2H) ; 5,14 (sl, 1H) ; 5,89 et 6,02 (sl, 2H) ; 6,09 et 6,21 (dl, 1H) ; 7,12 à 7,56 (m, 10H) ; 8,09 et 8,44 (dl, 1H).

### Exemple 12

On procède comme indiqué à l'exemple 4, sauf qu'au lieu d'utiliser l'amine obtenue à l'exemple 3, on utilise du cyano amino méthane du commerce.
On obtient ainsi un composé correspondant au (3S) 2-[1-oxo-3-[(phénylacétyl)amino]propyl]-3-[[(cyanoamino) carbonyl]-tétrahydro-1(2H)-Pyridazinecarboxylate de (phénylméthyle).
SM (Electrospray positif) m/z : [MH]⁺=492,3 [MNa]⁺= 514,3

### Exemple 13

On déprotège le composé obtenu à l'exemple 10, par hydrogénation comme indiqué à l'exemple 5.
On obtient le composé correspondant au (3S) N-cyano-2-[1-oxo-3-[(phénylacétyl)amino]propyl]-hexahydro-3-Pyridazine carboxamide.

### Spectre RMN du proton

Dans le CDCl₃, à 300 MHz, déplacements chimiques des pics en ppm et multiplicité :
1,56 (m, 2H) ; 1,66 et 2,46 (m, 2H) ; 2,26 et 3,10 (m, 2H) ; 2,72 et 2,99 (m, 2H); 3,20 et 4,08 (m, 2H) ;3,55 (sl, 2H) ; 3,78 et 4,09 (dd, 2H) ; 5,12 (dl, 1H) ; 6,04 (m, 1H) ; 7,19 à 7,40 (m, 5H) ; 7,97 (tl, 1H)

### Exemple 14

### Etape A

On procède comme indiqué à l'étape A de l'exemple 2, sauf qu'au lieu d'utiliser l'acide R₂OH obtenu à l'exemple 1, on utilise de l'acide 3-phényl propanoïque. On obtient ainsi un composé correspondant au (3S) 3-acétyl-2-[3-phényl-1-oxo-propyl]-tétrahydro-1(2H)-Pyridazine carboxylate de (phénylméthyle).

### Spectre RMN du proton

Dans le CDCl₃, à 300 MHz, déplacements chimiques des pics en ppm et multiplicité :
1,46 et 2,04 (m, 2H) ; 1,72 et 2,04 (m, 2H) ; 2,60 et 4,21 (m, 2H) ; 2,61 et 2,91 =(m, 4H) ; 3,54 (sl, 3H) ; 5,05 et 5,25 (d, 2H) ; 5,41 (dl, 1H) ; 7,10 à 7,33 (m, 10H)

### Etape B

On saponifie le produit obtenu à l'étape A comme indiqué à l'étape B de l'exemple 2 pour obtenir un acide.

### Spectre RMN du proton

Dans le CDCl₃, à 300 MHz, déplacements chimiques des pics en ppm et multiplicité :
1,5 (m, 2H) ; 1,90 (m, 2H) ; 2,6 (m, 2H) ; 3,0 (m, 2H); 4,1 (m, 1H) ; 5,3 (m, 2H) ; 7,35 (m, 5H) ; 7,4 (m, 5H).

### Etape C

On procède au couplage comme indiqué à l'exemple 4.
On obtient ainsi un composé correspondant au (3S) 3-[[[cyano(3-phénoxyphényl)méthyl]amino]carbonyl]-2-[3-phényl-1-oxo-propyl]-tétrahydro-1(2H)-Pyridazine carboxylate de (phénylméthyle).
SM (Electrospray positif) m/z : [MH]⁺=740,7

### Exemple 15

On déprotège le composé obtenu à l'exemple 14, par hydrogénation comme indiqué à l'exemple 5.
On obtient ainsi deux diastéréoisomères correspondant au (3S) N-[cyano(3-phénoxyphényl)méthyl]-2-(1-oxo-3-phénylpropyl)-hexahydro-3-Pyridazinecarboxamide.

### Spectre RMN du proton de l'un des diastéréoisomères

Dans le CDCl₃, à 300 MHz, déplacements chimiques des pics en ppm et multiplicité :
1,52 et 1,71 (m, 2H) ; 1,77 et 2,09 (m, 2H) ; 2,62 et 3,02 (m, 2H) ; 2,87 et 2,90 (m, 4H) ; 5,18 (sl, 1H) ; 6,03 (sl, 1H) ; 6,93 à 7,43 (m, 14H).

### Spectre RMN du proton de l'autre des diastéréoisomères

Dans le CDCl₃, à 300 MHz, déplacements chimiques des pics en ppm et multiplicité :
1,51 et 1,75 (m, 2H) ; 1,78 et 2,06 (m, 2H) ; 2,57 et 2,98 (m, 2H) ; 2,74 et 2,79 (m, 4H) ; 5,27 (sl, 1H) ; 5,96 (sl, 1H) ; 6,91 à 7,38 (m, 14H).

### Exemple 16

### Etape A

1 g (3,6 mmoles) du dérivé pyrazine utilisé dans l'exemple 2 sont mis en solution dans 20 ml de dichlorométhane. On ajoute à la solution 0.465 g (3,6 mmoles) de DIPEA et 0.616 g (3,6 mMoles) du chlorure d'acide 3-bromo propanoïque le mélange est conservé 12 H sous agitation à température ambiante. Le milieu réactionnel est concentré à sec, repris par 25 ml d'acétate d'éthyle, lavé par 25 ml d'une solution HCl 1N. La phase organique est séchée. L'huile obtenue est purifiée par chromatographie flash. On obtient 1,1 g du produit attendu correspondant au (3S) 2-(3-bromo-1-oxo-propyl)-tétrahydro-1,3(2H)-pyridazinedicarboxylate de 1-(phénylméthyle)-3-méthyle.
Le rendement correspondant est de 75%.

### Spectre RMN du proton

Dans le CDCl₃, à 300 MHz, déplacements chimiques des pics en ppm et multiplicité :
1,99 et 2,07 (m, 2H) ; 1,83 et 2,09 (m, 2H), 2,94 (m, 2H) ; 3,48 et 3,61 (m, 2H) ; 3,03 et 4,33 (m, 2H) ; 3,56 (s1, 3H), 5,09 et 5,27 (m, 2H) ; 5,40 (dd, 1H) ; 7,39 (m, 5H)

### Etape B

0,2 g (0.48 mmole) du composé obtenu à l'étape précédente est mis en solution dans 2 ml de DMF. On ajoute à la solution 0,421 mg (4,8 mmoles) de morpholine. Le mélange est conservé pendant 16 heures sous agitation. Le milieu est versé dans 25 ml d'eau puis extrait par 25 ml d'acétate d'éthyle.
La phase organique est séchée et concentrée. Le résidu obtenu est purifié par chromatographie flash. On obtient 91 mg du produit attendu.
Le rendement correspondant est de 50%.

### Spectre RMN du proton

Dans le CDCl₃, à 300 MHz, déplacements chimiques des pics en ppm et multiplicité :
1,53 et 1,92 (m, 2H) ; 1,78 et 1,92 (m, 2H) ; 2,3 à 2,7 (m, 4H) ; 3,52 (s, 3H) ; 3,38 (m, 4H) ; 3,57 (m, 4H) ; 5,18 (m, 1H) ; 5,04 et 5,2 (m,2H) ; 7,35 (m, 4H).

### Etape C

Le produit obtenu à l'étape B est saponifié comme indiqué à l'étape B de l'exemple 2.

### Spectre RMN du proton

Dans le DMSO, à 300 MHz, déplacements chimiques des pics en ppm et multiplicité :
1,43 (m, 2H) ; 1,30 et 1,97 (m, 2H) ; 2,16 (m, 4H) ; 2,82 et 4,07 (m, 2H) ; 3,43 (m, 4H) ; 4,40 (M, 4H) ; 4,66 (d, 1H) ; 4,85 et 5,16 (d 2H) ; 7,30 (m, 5H).

### Etape D

On procède comme indiqué à l'exemple 4, sauf qu'au lieu d'utiliser l'amine obtenue à l'exemple 3, on utilise du (cyano amino méthyl) benzène du commerce. On obtient ainsi un mélange 50/50 de deux diastéréoisomères correspondant au (3S)3-[[(cyanophénylméthyl)amino]carbonyl]-2-(4-morpholinyl carbonyl)-tétrahydro-1(2H)-Pyridazinedicarboxylate de (phénylméthyle).

### Spectre RMN du proton

Dans le DMSO, à 300 MHz, déplacements chimiques des pics en ppm et multiplicité :
1,5 (m, 2H) ; 1,81 (m, 2H) ; 2,52 (m, 2H); 2,28 (m, 2H) ; 3,17 et 4,09 (m, 2H) ; 3,49 (m, 2H) ; 5,05 et 5,21 (m, 2H) ; 6,07 (m, 1H) ; 6,97 à 8,87 (m, 14H) ; 8,87 (s1, 1H).

### Exemple 17

On procède comme dans l'exemple 16, en remplaçant, à l'étape B, le chlorure d'acide 3-bromo propanoïque par le chlorure d'acide de la 4-morpholine et à l'étape D, le (cyano amino méthyl) benzène du commerce par l'amine obtenue à l'exemple 3.
On obtient ainsi le composé correspondant au (3S) 3-[[[cyano(3-phénoxyphényl)méthyl]amino]-2-[(4-morpholinyl) carbonyl]-]-tétrahydro-1(2H)-Pyridazinecarboxylate de (phénylméthyle).

### Spectre RMN du proton

Dans le CDCl₃, à 300 MHz, déplacements chimiques des pics en ppm et multiplicité :
1,53 et 1,70 (m, 2H) ; 1,80 et 1,93 (m, 2H) ; 3,12 et 3,24 (m, 4H) ; 3,40 (m, 4H) ; 4,34 (s1, 1H) ; 5,05 et 5,17 (m, 2H) ; 6,10 (sl, 1H) ; 6,98 à 7,49 (m, 14H)

### Exemple 18

On déprotège le composé obtenu à l'exemple 17, par hydrogénation comme indiqué à l'exemple 5.
On obtient ainsi un composé correspondant au (3S) N-[cyano (3-phénoxyphényl)méthyl]-2- [(4-morpholinyl) carbonyl]-hexahydro-3-Pyridazinecarboxamide.

### Spectre RMN du proton

Dans le CDCl₃, à 300 MHz, déplacements chimiques des pics en ppm et multiplicité :
1,57 et 1,76 (m, 2H) ; 1,83 et 2,14 (m, 2H) ; 2,77 et 2,98 (m, 2H) ; 3,31 et 3,48 (m, 4H) ; 3,62 (m, 4H) ; 4,52 (m, 1H) ; 6,09 (m, 1H) ; 6,95 à 7,4 (m, 9H)

### Exemple 19

On procède comme dans l'exemple 16, en remplaçant, à l'étape B, le chlorure d'acide 3-bromo propanoïque par le chlorure d'acide de la 4-morpholine.
On obtient ainsi un composé correspondant au (3S)3-[[(cyanophénylméthyl)amino]carbonyl]-2-(4-morpholinyl carbonyl)-tétrahydro-1(2H)-Pyridazinedicarboxylate de (phénylméthyle).

### Spectre RMN du proton

Dans le CDCl₃, à 300 MHz, déplacements chimiques des pics en ppm et multiplicité :
1,60 et 1,75 (m, 2H) ; 1,85 et 2,35 (m, 2H) ; 3,64 et 3,50 (m, 4H) ; 3,29 et 3,43 (m, 4H) ; 3,36 et 3,96 (m, 1H) ; 3,38 et 4,07 (m, 1H) ; 4,66 et 4,92 (m, 1H) ; 5,23 et 5,27 (m, 1H) ; 6,04 (m, 1H) ; 7,4 (m, 10H).

### Exemple 20

On déprotège le composé obtenu à l'exemple 19, par hydrogénation comme indiqué à l'exemple 5.

On obtient ainsi un composé correspondant au (3S) N-[cyanophénylméthyl]-2-[(4-morpholinyl)carbonyl]-hexahydro-3-Pyridazinecarboxamide.

### Spectre RMN du proton

Dans le CDCl₃, à 300 MHz, déplacements chimiques des pics en ppm et multiplicité :
1,83 à 3,30 (m, 6H) ; 3,35 (m, 4H) ;3,67 (m, 4H) ; 4,7 (m, 1H) ; 6,14 (m, 1H) ; 7,4 (m, 5H)

### Exemple 21

On procède comme dans l'exemple 16, en remplaçant, à l'étape B, le chlorure d'acide 3-bromo propanoïque par le chlorure d'acide de la 4-morpholine et à l'étape D, le (cyano amino méthyl) benzène du commerce par du cyano amino méthane.
On obtient ainsi un composé correspondant au (3S) 3-[[[cyano(3-phénoxyphényl)méthyl]amino]-2-[(4-morpholinyl) carbonyl]-]-tétrahydro-1(2H)-Pyridazine carboxylate de (phénylméthyle).
SM (Electrospray positif) m/z : [MH]⁺=415

### Exemple 22

On procède comme dans l'exemple 16, en remplaçant, à l'étape B, le chlorure d'acide 3-bromo propanoïque par le chlorure d'acide de la 4-morpholine et à l'étape D, le (cyano amino méthyl) benzène du commerce par du 1-cyano 1-amino cyclohexane.
On obtient ainsi un composé correspondant au (3S) 3-[[(1-cyanocyclohexyl)amino]carbonyl]-2-[(4-morpholinyl) carbonyl]-tétrahydro-1(2H)-Pyridazinecarboxylate de (phénylméthyle).

### Spectre RMN du proton

Dans le DMSO, à 300 MHz, déplacements chimiques des pics en ppm et multiplicité :
1,20 à 2,15 (m, 12H) ; 1,76 et 2,02 (m, 2H) ; 3,17 et 3,33 (m, 4H) ; 3,51 (m, 4H) ; 3,50 et 3,92 (m, 2H) ; 4,33 (sl, 1H) ; 5,0 à 5,25 (m, 2H) ; 7,36 (m, 5H)

### Exemple 23

On déprotège le composé obtenu à l'exemple 22, par hydrogénation comme indiqué à l'exemple 5.
On obtient ainsi un composé correspondant au (3S) N-(1-cyanocyclohexyl]-2-[(4-morpholinyl)carbonyl]-hexahydro-3-Pyridazinecarboxamide.

### Spectre RMN du proton

Dans le CDCL3, à 300 MHz, déplacements chimiques des pics en ppm et multiplicité :
1.32 à 1.62 (m, 2H) ; 1.47 à 1.62 (m, 2H) ; 1.62 à 1.97 (m, 2H) ; 1.67 (m, 2H) ; 1.72 à 2.31 (m,3H) ; 1.83 à 2.24 (m, 2H) ; 1.83 à 2.24 (m, 2H) ; 2.92 à 3.14 (m, 2H) ; 3.43 à 3.56 (m, 4H) ; 3.70 (m, 4H) ; 4.56 (sl, 1H) ;

### Exemple 24

### Etape A

On déprotège le composé obtenu à l'étape A de l'exemple 16 comme indiqué à l'exemple 5, pour obtenir le composé correspondant au (3S) 2-[(4-morpholinyl)carbonyl]-tétrahydro-1(2H)-pyridazinecarboxylate de méthyle.

### Etape B

A une solution de 5 ml de DMF contenant 100 mg (0,002 mol) de NaH, on introduit goutte à goutte 200 mg (0,8 mmol) d'ester en solution dans 5 ml de DMF. à 0°C. Après retour du mélange à température ambiante, on ajoute 320 ml (5 mmol) d'iodure de méthyle. Le milieu réactionnel est conservé 15 H sous agitation à 100 °C. Le milieu est versé dans 25 ml d'eau puis extrait par 25 ml d'acétate d'éthyle, la phase organique est séchée puis concentrée. Après purification par chromatographie flash, on obtient 125 mg d'une huile correspondant au (3S) 2-[(4-morpholinyl)carbonyl]-1-méthyl-tétrahydro-1(2H)-pyridazinecarboxylate de méthyle.
Le rendement correspondant est de 60%.

### Spectre RMN du proton

Dans le DMSO, à 300 MHz, déplacements chimiques des pics en ppm et multiplicité :
1,38, 1,76 et 1,84 (sl, 4H) ; 2,50 (m, 3H) ; 2,81 et 2,91 (m, 2H) ; 3,33 (m, 4 H) ; 3,53 (m, 4H) ; 3,56 (s, 3H) ; 4,04 (sl, 1H)

### Etape C

Le produit obtenu à l'étape B est saponifié comme indiqué à l'Etape B de l'exemple 2.

### Spectre RMN du proton

Dans le DMSO, à 300 MHz, déplacements chimiques des pics en ppm et multiplicité :
1,51 et 1,61 (m, 1H) ; 1,83 (m, 2H) ; 2,53 (s, 3H) ; 2,86 (m, 2H) ; 3,36 (m, 4H) ; 3,55 (m, 4H) ; 4,02 (t, 1H)

### Etape D

On procède avec le produit obtenu à l'étape C comme indiqué à l'Etape D de l'exemple 16 en remplaçant le (cyano amino méthyl) benzène du commerce par l'amine obtenue à l'exemple 3.

On obtient ainsi le produit correspondant au (3S) N-[cyano(3-phénoxyphényl)méthyl]-1-méthyl-2-[(4-morpholinyl)carbonyl]-hexahydro-3-Pyridazinecarboxamide.
SM (Electrospray positif) m/z : [MH]⁺=464

### Exemple 25

### Etape A

Cette étape est identique à celle de l'exemple 24.

### Etape B

A une solution de 3 ml de DMF contenant 200 mg (0.8 mmol) d'ester on ajoute 400 mg de carbonate de potassium (2.72 mmol). Le mélange est porté à 100°C. On introduit 322 ml (2.4 mmol) de bromure de benzyle. On conserve le mélange 15 heures sous agitation à 100°C. Le milieu est versé dans 25 ml d'eau puis extrait par 25 ml d'acétate d'éthyle. La phase organique est séchée puis concentrée. Après purification par chromatographie flash, on récupère 158 mg d'une huile correspondant au (3S) 2-[(4-morpholinyl)carbonyl]-1-phénylméthyl-tétrahydro-1(2H)-pyridazinecarboxylate de méthyle.
Le rendement correspond est de 59 %.
SM (Electrospray positif) m/z : [MH]+: 348

### Etape C:

Le produit obtenu à l'étape C est saponifié comme indiqué à l'étape B de l'exemple 2 et on obtient l'acide carboxylique correspondant.
Le rendement correspondant est de 91 %.
SM (Electrospray positif) m/z : [MH]+: 334

### Etape D:

On procède avec le produit obtenu à l'étape C comme indiqué à l'étape D de l'exemple 16 en remplaçant le (cyano amino méthyl) benzène du commerce par l'amine obtenue à l'exemple 3.
On obtient ainsi le produit (mélange de 2 diastéréoisomères) correspondant au (3S) N-[cyano(3-phénoxyphényl)méthyl]-1-(phénylméthyl)-2-[(4-morpholinyl)carbonyl]-hexahydro-3-Pyridazinecarboxamide. Les 2 diastéréoisomères sont séparés par chromatographie flash.
On obtient 28 mg de l'iso A et 16.5 mg de l'iso B (rendement global= 27%).

### Spectre RMN du proton

Dans le DMSO, à 300 Mhz, déplacements chimiques des pics en PPM et multiplicité :
**Premier Isomère :** 1.63 à 1.77 (m, 2H) ; 1.94 (m, 2H) ; 2.78 (m, 2H) ; 3.29 (m, 4H) ; 3.51 (t, 4H) ; 3.84 (m, 1H) ; 3.91 (m, 2H) ; 3.99(m, 1H) ; 4.20 (m, 1H) ; 6.08 et 6.16 (d, 1H) ; 6.98 à 7.42 (m, 14H) ; 8.91 et 9.00 (sl,1H).
**Deuxième isomère** : 1.62 à 1.77 (m, 2H) ; 1.94 (m, 2H) ; 2.78 (m, 2H) ; 3.30 (m, 4H) ; 3.51 (m, 4H) ; 3.84 (m, 1H) ; 3.92 (m, 2H) ; 4.01 (m, 1H) ; 4.20 (m, 1H) ; 6.08 et 6.16 (d, 2H) ; 6.98 à 7.42 (m, 14H) ; 8.91 et 9.00 (sl,1H).

### Exemple 26

### Etude pharmacologique des produits de l'invention

### Etude de l'inhibition de la Cathepsine K

Les produits à tester (10 mM) sont dilués à 1 mM en DMSO et répartis dans des plaques 96 puits polystyrène Nunc à raison de 2 µl par puits. La colonne 12 de la plaque est réservée pour les contrôles et reçoit donc 1 µl de DMSO (sans produits) par puits. Les plaques sont conservées à -80°C et décongelées le jour de l'expérience.
Les produits sont dilués à 50 µM par addition de 38 µl de tampon réaction : acétate de sodium 100 mM, EDTA 5 mM, DTT 1 mM, pH 5,5. L'addition ainsi que tous les pipetages suivant sont réalisés par un pipeteur 96 cônes CybiWell. Après mélange des solutions, chaque produit est transféré dans 2 puits (duplicates) d'une plaque 384 puits noire Greiner à raison de 10 µl par puits. On peut donc tester 2 plaques 96 dans une plaque 384.
Une solution de substrat à 50 µM, Z-Val-arg-AMC (Calbiochem), est préparée dans le tampon réaction. Le substrat, est ensuite distribué dans tous les puits de la plaque 384 (20 µl par puits).
Une solution de Cathepsine K à 12,5 ng/ml est préparée dans le tampon réaction et distribuée dans tous les puits de la plaque 384 (20 µl par puits) exceptés les 16 puits servant de contrôles 100 % d'inhibition (colonne 23 et 24, lignes I à P) qui recevront 20 µl de tampon sans enzyme. Les contrôles 100 % d'inhibition sont réalisés dans les colonnes 23 et 24, lignes A à H qui ne contiennent pas de produits.
Les plaques sont ensuite incubées 2H à température ambiante, puis lues sur Fluoroskan (Labsystems) :
excitation 390 nm ; émission 460 nm

Les concentrations finales de chacun des réactifs sont :
Produits 10 µM, Substrat 20 µM, enzyme 5 ng/ml.
Les % d'inhibition pour chacun des produits sont calculés en utilisant les points à 0 et 100 % d'inhibition de chaque plaque comme références. Les produits présentant une inhibition significative sont ensuite retestés sur une gamme de concentration allant de 50 à 0,5µM pour déterminer une CI50.

### Résultats

Les CI50 trouvées pour certains produits sont données dans le tableau I ci-après, en micromoles :

**TABLEAU I**

| **Exemple** | **CI**_{**50**} **(µM)** |
|---|---|
| 4 | 1-10 |
| 5,6 | <1 |
| 7 | 1-10 |
| 8 | 1-10 |
| 9 | 1-10 |
| 10 | 1-10 |
| 11 | 1-10 |
| 12 | 1-10 |
| 13 | 1-10 |
| 14 | 1-10 |
| 15 | <1 (2 valeurs) |

### Exemple 25

### Composition pharmaceutique

On a préparé des comprimés répondant à la formule suivante :

| | |
|---|---|
| Composé de l'exemple 1 | 500 mg |
| Excipient pour un comprimé terminé à | 1 g |
| (détail de l'excipient : lactose, talc, amidon, stéarate de magnésium). | |

## Revendications

1. Produits de formule générale (I) : 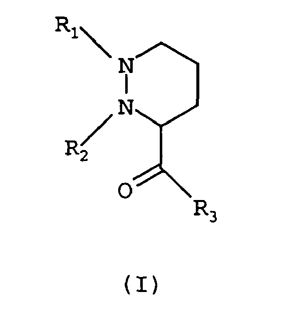 dans laquelle :
R₁ représente un atome d'hydrogène ou un groupement alkyle linéaire ou ramifié ayant de 1 à 6 atomes de carbone, COR, COOR, R étant choisi dans le groupe constitué par un radical alkyle linéaire ou ramifié renfermant de 1 à 6 atomes de carbone, éventuellement substitué par un radical pyridyle ou carbamoyle, un radical -CH₂-alkényle linéaire ou ramifié renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle linéaire ou ramifié renfermant de 1 à 6 atomes de carbone, alkoxy linéaire ou ramifié renfermant de 1 à 6 atomes de carbone ou par 1 à 3 atomes d'halogène,
R₂ représente un groupement répondant à la formule (II) suivante : 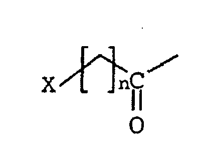 dans laquelle :
n vaut 0,1,2 ou 3 ; une double liaison pouvant éventuellement être présente lorsque n vaut 2 ou 3;
X est l'un des groupements:
groupement hétérocyle monocyclique ou bicyclique saturé ou insaturé; ou
un groupement aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle linéaire ou ramifié renfermant de 1 à 6 atomes de carbone, alkoxy linéaire ou ramifié renfermant de 1 à 6 atomes de carbone ou par 1 à 3 atomes d'halogène ; ou
un groupement NR₄R₅, R₄ étant un groupement alkyle linéaire ou ramifié ayant de 1 à 6 atomes de carbone ou un groupement COR, CONHR, CSNHR ou SO₂R, R ayant la signification donnée précédemment et R₅ étant un atome d'hydrogène ou un radical alkyle linéaire ou ramifié ayant de 1 à 6 atomes de carbone ; ou
un groupement COR, R ayant la signification donnée précédemment ;
R₃ est un groupement de formule -Y-(CH₂)ₘ-C(CN)R₆R₇, dans laquelle :
Y est un atome d'oxygène ou un groupement -N(R₈)-, R₈ étant un atome d'hydrogène ou un groupement alkyle linéaire ou ramifié ayant de 1 à 6 atomes de carbone,
m vaut 0,1,2 ou 3,
R₆ est un atome d'hydrogène, un groupement alkyle linéaire ou ramifié ayant de 1 à 6 atomes de carbone ou un groupement aryle ou alkaryle, le noyau du radical aryle ou aralkyle étant éventuellement substitué par un radical OH, NH₂, NO₂, alkyle linéaire ou ramifié renfermant de 1 à 6 atomes de carbone, alkoxy linéaire ou ramifié renfermant de 1 à 6 atomes de carbone, aryloxy renfermant de 7 à 11 atomes de carbone, ce groupement aryloxy étant lui-même éventuellement substitué par 1 à 3 halogènes,
R₇ est un atome d'hydrogène ou un groupement alkyle linéaire ou ramifié ayant de 1 à 6 atomes de carbone,
ou R₆ et R₇ pouvant former ensemble un cycle saturé à 6 chaînons ;
lesdits produits de formule (I) étant sous toutes les formes isomères possibles, racémiques, énantiomères et diastéréo-isomères ;
ainsi que les sels d'addition avec les acides minéraux et organiques ou avec les bases minérales et organiques de ces produits.

2. Composé selon la revendication 1, **caractérisé en ce que** R₁ est un atome d'hydrogène, un groupement méthyle, benzyle, -COO-benzyle ou -CO-méthylène-benzyle.

3. Composé selon la revendication 1 ou la revendication 2, **caractérisé en ce que** n est égal à 0 ou 2.

4. Composé selon l'une des revendications 1 à 3, **caractérisé en ce que** X est le groupement NR₄R₅, et R₅ est un atome d'hydrogène.

5. Composé selon l'une des revendications 1 à 3, **caractérisé en ce que** X est un groupement phényle ou NHCO-benzyle.

6. Composé selon l'une des revendications 1 à 3, **caractérisé en ce que** X est le groupement

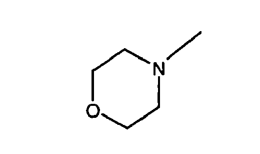

7. Composé selon l'une des revendication 1 à 6, **caractérisé en ce que** R₈ est un atome d'hydrogène.

8. Composé selon l'une des revendications 1 à 7, **caractérisé en ce que** R₇ est un atome d'hydrogène.

9. Composé selon l'une des revendications 1 à 8, **caractérisé en ce que** R₆ est un atome d'hydrogène.

10. Composé selon l'une des revendications 1 à 8, **caractérisé en ce que** R₆ est le groupement phényle,
-C₆H₄-O-C₆H₅ ou -C₂H₄-O-C₆H₄Br.

11. Composé selon l'une des revendications 1 à 10, **caractérisé en ce que** m est égal à 0 ou 2.

12. Composé selon la renvendication 1, choisi parmi les composés suivants:
(3S) 2-[3-[(phénylacétyl)amino]-1-oxopropyl]-N-[cyano(3-phénoxyphényl)méthyl]-hexahydro-3-Pyridazinecarboxamide; et
(3S) N-[cyano(3-phénoxyphényl)méthyl]-2-(1-oxo-3-phénylpropyl)-hexahydro-3-Pyridazinecarboxamide.

13. Composé selon l'une quelconque des revendications 1 à 12, présentant la stéréochimie suivante:

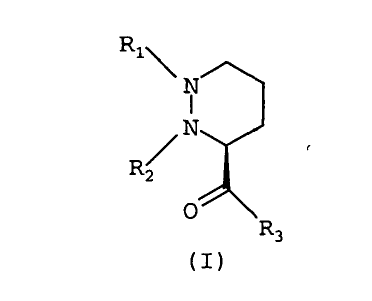

14. Composé selon l'une des revendications 1 à 13, pour son utilisation à titre de médicament.

15. Composé selon la revendication 14, pour son utilisation à titre de médicament destiné à la prévention ou au traitement de maladies dans lesquelles des enzymes métaboliques choisies parmi les protéases et des kinases sont impliquées.

16. Composé selon la revendication 14 ou 15, pour son utilisation à titre de médicament destiné à la prévention ou au traitement de maladies dans lesquelles est impliquée la cathépsine K.

17. Composé selon la revendication 15 ou 16, les maladies à prévenir ou à traiter étant choisies dans le groupe de maladies consistant en maladies cardiovasculaires, cancers, maladies du système nerveux central, maladies inflammatoires, maladies infectieuses et maladies de l'os.

18. Composé selon la revendication 15 ou 16, les maladies à prévenir ou à traiter sont l'ostéoporose, l'hypercalcémie, l'ostéopénie, maladies gingivales, arthrite, maladie de Paget, cancers osseux.

19. Composition pharmaceutique contenant, à titre de principe actif, au moins un composé selon l'une des revendications 1 à 18, en association avec un support pharmaceutiquement acceptable.

20. Utilisation d'un composé selon l'une des revendications 1 à 18, pour la préparation d'un médicament destiné à la prévention ou au traitement de maladies dans lesquelles des enzymes métaboliques choisies parmi les protéases et des kinases sont impliquées.

21. Procédé de préparation d'un produit selon l'une des revendications 1 à 13, **caractérisé en ce qu'**il comporte l'étape de réaction d'un produit de formule (IV) 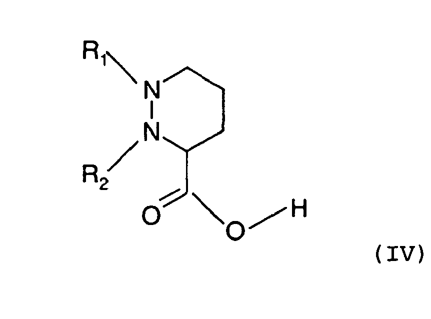 dans laquelle R₁ et R₂ ont la même signification que dans l'une des revendications 1 à 13,
avec un amino-nitrile ou cyano-hydrine de formule HR₃, dans laquelle R₃ a la même signification que dans l'une des revendications revendication 1 à 13, pour obtenir un produit de formule (I).

22. Procédé de préparation d'un produit selon l'une des revendications 1 à 13, **caractérisé en ce qu'**il comporte :
1) une étape au cours de laquelle on fait réagir un produit de formule (II) 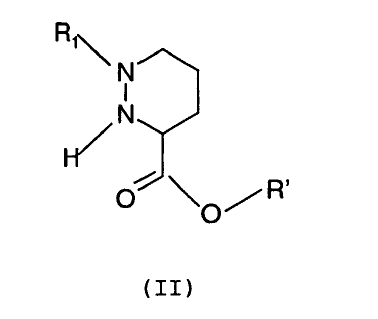 avec un chlorure d'acide de formule R₂Cl, dans laquelle R₁ et R₂ ont les mêmes significations que dans l'une des revendications 1 à 13 et R' est un groupement alkyle linéaire ou ramifié ayant de 1 à 6 atomes de carbone, pour obtenir le produit de formule (III); 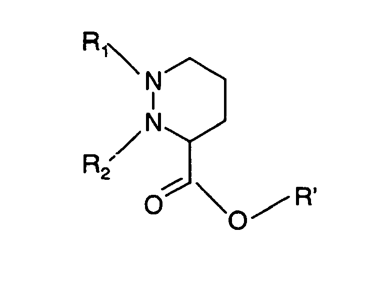
2) une étape au cours de laquelle on hydrolyse le produit de formule (III) obtenu à l'étape 1) en le produit de formule (IV); 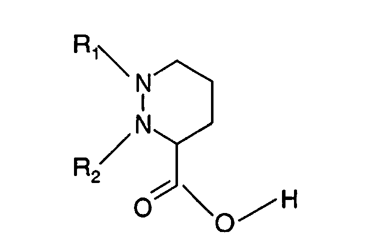
3) une étape au cours de laquelle on fait réagir le produit de formule (IV) obtenu à l'étape 2) avec un amino-nitrile ou cyano-hydrine de formule HR₃, dans laquelle R₃ a la même signification que dans l'une des revendications 1 à 13, pour obtenir un produit de formule (I).

23. Procédé selon la revendication 21 ou 22, **caractérisé en ce qu'**il comprend une étape d'hydrogénolyse du produit final.

24. Procédé selon l'une des revendications 21 à 23, **caractérisé en ce qu'**il comprend une ou plusieurs des réactions optionnelles suivantes, dans un ordre approprié :
- protection des fonctions réactives,
- déprotection des fonctions réactives,
- estérification,
- saponification,
- amidification,
- acylation,
- sulfonylation;
- alkylation;
- introduction d'une double liaison;
- formation d'un groupe urée;
- réduction d'acides carboxyliques;
- déshydratation d'amide en nitrile;
- salification;
- échange d'ions;
- dédoublement ou séparation de diastéréoisomères.

## Claims

1. Products of formula (I): 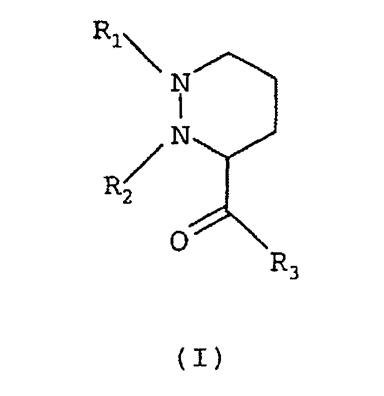 in which:
R₁ represents a hydrogen atom or a linear or branched alkyl group containing from 1 to 6 carbon atoms, COR or COOR, R being chosen from the group consisting of a linear or branched alkyl radical containing from 1 to 6 carbon atoms, optionally substituted with a pyridyl or carbamoyl radical, a linear or branched -CH₂-alkenyl radical containing in total from 3 to 9 carbon atoms, an aryl radical containing from 6 to 10 carbon atoms or an aralkyl radical containing from 7 to 11 carbon atoms, the nucleus of the aryl or aralkyl radical being optionally substituted with an OH radical, NH₂ radical, NO₂ radical, linear or branched alkyl radical containing from 1 to 6 carbon atoms or linear or branched alkoxy radical containing from 1 to 6 carbon atoms, or with 1 to 3 halogen atoms,
R₂ represents a group corresponding to formula (II) below: 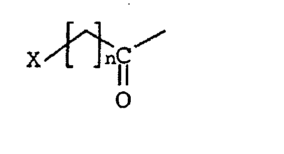 in which
n is 0, 1, 2 or 3; a double bond possibly being present when n is 2 or 3;
X is one of the following groups:
saturated or unsaturated monocyclic or bicyclic heterocyclic group; or
an aryl group containing from 6 to 10 carbon atoms or an aralkyl group containing from 7 to 11 carbon atoms, the nucleus of the aryl or aralkyl radical being optionally substituted with an OH radical, NH₂ radical, NO₂ radical, linear or branched alkyl radical containing from 1 to 6 carbon atoms or linear or branched alkoxy radical containing from 1 to 6 carbon atoms, or with 1 to 3 halogen atoms; or a group NR₄R₅, R₄ being a linear or branched alkyl group containing from 1 to 6 carbon atoms or a group COR, CONHR, CSNHR or SO₂R, R having the meaning given above and R₅ being a hydrogen atom or a linear or branched alkyl radical containing from 1 to 6 carbon atoms; or
a group COR, R having the meaning given above
R₃ is group of formula -Y-(CH₂)ₘ-C(CN)R₆R₇, in which:
Y is an oxygen atom or a group -N(R₈)-,
R₈ being a hydrogen atom or a linear or branched alkyl group containing from 1 to 6 carbon atoms,
m is 0, 1, 2 or 3,
R₆ is a hydrogen atom, a linear or branched alkyl group containing from 1 to 6 carbon atoms or an alkyl or aralkyl group, the nucleus of the aryl or aralkyl radical being optionally substituted with an OH radical, NH₂ radical, NO₂ radical, linear or branched alkyl radical containing from 1 to 6 carbon atoms, linear or branched alkoxy radical containing from 1 to 6 carbon atoms, or aryloxy radical of 7 to 11 carbon atoms, this aryloxy group being itself optionally substituted with 1 to 3 halogen atoms,
R₇ is a hydrogen atom or a linear or branched alkyl group containing from 1 to 6 carbon atoms,
or R₆ and R₇ together possibly forming a saturated 6-membered ring;
said products of formula (I) being in any possible racemic, enantiomeric or diastereoisomeric isomer form; and also the addition salts of these products with mineral and organic acids or with mineral and organic bases.

2. Compound according to Claim 1, **characterized in that** R₁ is a hydrogen atom or a methyl, benzyl, -COO-benzyl or -CO-methylenebenzyl group.

3. Compound according to Claim 1 or Claim 2, **characterized in that** n is equal to 0 or 2.

4. Compound according to one of Claims 1 to 3, **characterized in that** X is an NR₄R₅ group and R₅ is a hydrogen atom.

5. Compound according to one of Claims 1 to 3, **characterized in that** X is a phenyl or -NHCO-benzyl group.

6. Compound according to one of Claims 1 to 3, **characterized in that** X is a group

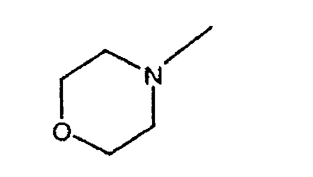

7. Compound according to one of Claims 1 to 6, **characterized in that** R₈ is a hydrogen atom.

8. Compound according to one of Claims 1 to 7, **characterized in that** R₇ is a hydrogen atom.

9. Compound according to one of Claims 1 to 8, **characterized in that** R₆ is a hydrogen atom.

10. Compound according to one of Claims 1 to 8, **characterized in that** R₆ is a phenyl, -C₆H₄-O-C₆H₅ or -C₂H₄-O-C₆H₄Br group.

11. Compound according to one of Claims 1 to 10, **characterized in that** m is equal to 0 or 2.

12. Compound according to Claim 1, chosen from the following compounds:
(3S)-2-[3-[(phenylacetyl)amino]-1-oxopropyl]-N-[cyano-(3-phenoxyphenyl)methyl]hexahydro-3-pyridazine-carboxamide, and
(3S)-N-[cyano(3-phenoxyphenyl)methyl]-2-[1-oxo-3-phenylpropyl)hexahydro-3-pyridazinecarboxamide.

13. Compound according to any one of Claims 1 to 12, having the following stereochemistry:

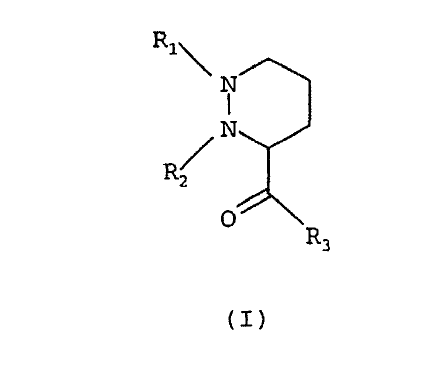

14. Compound according to one of claims 1 to 13, for its use as a medicinal product.

15. Compound according to Claim 14, for its use as a medicinal product for preventing or treating diseases in which metabolic enzymes chosen from proteases and kinases are involved.

16. Compound according to Claim 14 or 15, for its use as a medicinal product for preventing or treating diseases in which cathepsin K is involved.

17. Compound according to Claim 15 or 16, the diseases to be prevented or treated being chosen from the group of diseases consisting of cardiovascular diseases, cancers, diseases of the central nervous system, inflammatory diseases, infectious diseases and bone diseases.

18. Compound according to Claim 15 or 16, the diseases to be prevented or treated being osteoporosis, hypercalcaemia, osteopenia, gingival diseases, arthritis, Paget's disease and bone cancers.

19. Pharmaceutical composition containing, as active principle, at least one compound according to one of Claims 1 to 18, in combination with a pharmaceutically acceptable support.

20. Use of a compound according to one of Claims 1 to 18, for the preparation of a medicinal product for preventing or treating diseases in which metabolic enzymes chosen from proteases and kinases are involved.

21. Process for preparing a product according to one of Claims 1 to 13, **characterized in that** it includes the step of reaction of a product of formula (IV) 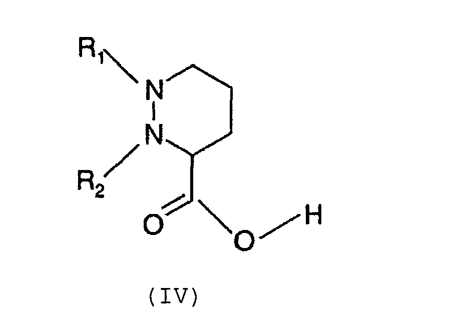 in which R₁ and R₂ have the same meaning as in one of Claims 1 to 13,
with an aminonitrile or cyanohydrin of formula HR₃, in which R₃ has the same meaning as in one of Claims 1 to 13, to obtain a product of formula (I).

22. Process for preparing a product according to one of Claims 1 to 13, **characterized in that** it includes:
1) a step during which a product of formula (II) 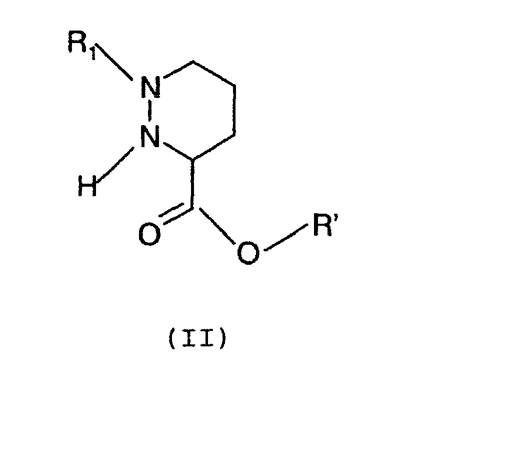 is reacted with an acid chloride of formula R₂Cl, in which R₁ and R₂ have the same meanings as in one of Claims 1 to 13 and R' is a linear or branched alkyl group containing from 1 to 6 carbon atoms, to obtain the product of formula (III): 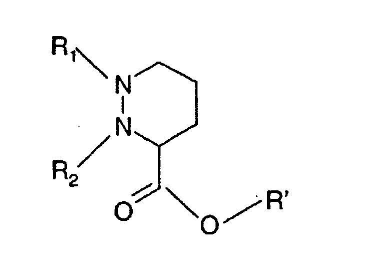
2) a step during which the product of formula (III) obtained in step 1) is hydrolysed to the product of formula (IV): 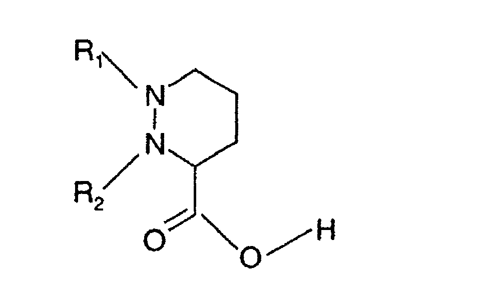
3) a step during which the product of formula (IV) obtained in step 2) is reacted with an aminonitrile or cyanohydrin of formula HR₃, in which R₃ has the same meaning as in one of claims 1 to 13, to obtain a product of formula (I).

23. Process according to Claim 21 or 22, **characterized in that** it includes a step of hydrogenolysis of the final product.

24. Process according to one of Claims 21 to 23,
**characterized in that** it includes one or more of the following optional reactions, in an appropriate order:
- protection of reactive functions;
- deprotection of reactive functions;
- esterification;
- saponification;
- amidation;
- acylation;
- sulfonylation;
- alkylation;
- introduction of a double bond;
- formation of a urea group;
- reduction of carboxylic acids;
- dehydration of amide to nitrile;
- salification;
- ion exchange;
- resolution or separation of diastereoisomers.

## Patentansprüche

1. Produkte der allgemeinen Formel (I) 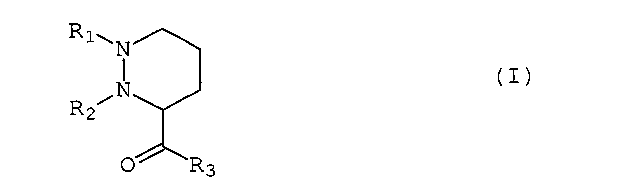 in der
R₁ ein Wasserstoffatom oder eine lineare oder verzweigte Gruppe Alkyl mit 1 bis 6 Kohlenstoffatomen, COR, COOR, worin R aus der Gruppe gewählt wird, die gebildet wird durch einen linearen oder verzweigten Rest Alkyl mit 1 bis 6 Kohlenstoffatomen, gegebenenfalls substituiert durch einen Rest Pyridyl oder Carbamoyl, einen linearen oder verzweigten Rest -CH₂-Alkenyl mit insgesamt 3 bis 9 Kohlenstoffatomen, Aryl, umfassend 6 bis 10 Kohlenstoffatome oder Aralkyl, umfassend 7 bis 11 Kohlenstoffatome, darstellt, wobei der Kern des Restes Aryl oder Aralkyl gegebenenfalls substituiert ist durch einen Rest OH, NH₂, NO₂, lineares oder verzweigtes Alkyl mit 1 bis 6 Kohlenstoffatomen, lineares oder verzweigtes Alkoxy mit 1 bis 6 Kohlenstoffatomen oder durch 1 bis 3 Halogenatome,
R₂ eine Gruppe darstellt, die der folgenden allgemeinen Formel (II) entspricht: 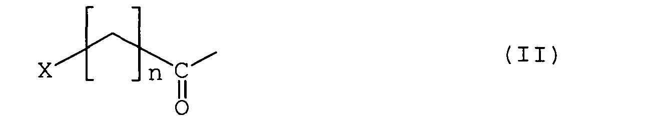 in der
n den Wert 0, 1, 2 oder 3 besitzt; wobei gegebenenfalls eine Doppelbindung anwesend sein kann, wenn n gleich 2 oder 3 ist;
X eine der folgenden Gruppen darstellt:
monocyclische oder bicyclische, gesättigte oder ungesättigte Gruppe Heterocyclyl; oder
eine Gruppe Aryl, umfassend 6 bis 10 Kohlenstoffatome oder Aralkyl, umfassend 7 bis 11 Kohlenstoffatome, wobei der Kern des Restes Aryl oder Aralkyl gegebenenfalls substituiert ist durch einen Rest OH, NH₂, NO₂, lineares oder verzweigtes Alkyl mit 1 bis 6 Kohlenstoffatomen, lineares oder verzweigtes Alkoxy mit 1 bis 6 Kohlenstoffatomen oder durch 1 bis 3 Halogenatome; oder
eine Gruppe NR₄R₅, in der R₄ eine lineare oder verzweigte Gruppe Alkyl mit 1 bis 6 Kohlenstoffatomen oder eine Gruppe COR, CONHR, CSNHR oder SO₂R ist, worin R die vorstehend angegebene Bedeutung besitzt, und R₅ ein Wasserstoffatom oder ein linearer oder verzweigter Rest Alkyl mit 1 bis 6 Kohlenstoffatomen; oder eine Gruppe COR ist, worin R die vorstehend angegebene Bedeutung besitzt;
R₃ eine Gruppe der Formel -Y-(CH₂)ₘ-C(CN)R₆R₇ darstellt, in der Y ein Sauerstoffatom oder eine Gruppe -N(R₈)- ist, worin R₈ ein Wasserstoffatom oder eine lineare oder verzweigte Gruppe Alkyl mit 1 bis 6 Kohlenstoffatomen bedeutet,
m den Wert 0, 1, 2 oder 3 besitzt;
R₆ ein Wasserstoffatom, eine lineare oder verzweigte Gruppe Alkyl mit 1 bis 6 Kohlenstoffatomen oder eine Gruppe Aryl oder Alkaryl darstellt, wobei der Kern des Restes Aryl oder Aralkyl gegebenenfalls substituiert ist durch einen Rest OH, NH₂, NO₂, lineares oder verzweigtes Alkyl mit 1 bis 6 Kohlenstoffatomen, lineares oder verzweigtes Alkoxy mit 1 bis 6 Kohlenstoffatomen, Aryloxy mit 7 bis 11 Kohlenstoffatomen, wobei diese Gruppe Aryloxy gegebenenfalls substituiert ist durch 1 bis 3 Halogene,
R₇ ein Wasserstoffatom oder eine lineare oder verzweigte Gruppe Alkyl mit 1 bis 6 Kohlenstoffatomen darstellt,
oder R₆ und R₇ zusammen einen gesättigten Ring mit 6 Kettengliedern bilden können;
wobei die genannten Produkte der Formel (I) in allen möglichen isomeren Formen, Racematen, Enantiomeren und Diastereoisomeren vorliegen können,
sowie die Additionssalze dieser Produkte mit Mineralsäuren und organischen Säuren oder mit Mineralbasen und organischen Basen.

2. Verbindung nach Anspruch 1, **dadurch gekennzeichnet, daß** R₁ ein Wasserstoffatom, eine Gruppe Methyl, Benzyl, -COO-Benzyl oder -CO-Methylen-benzyl ist.

3. Verbindung nach Anspruch 1 oder 2, **dadurch gekennzeichnet, daß** n gleich 0 oder 2 ist.

4. Verbindung nach einem der Ansprüche 1 bis 3, **dadurch gekennzeichnet, daß** X die Gruppe NR₄R₅ darstellt und R₅ ein Wasserstoffatom ist.

5. Verbindung nach einem der Ansprüche 1 bis 3, **dadurch gekennzeichnet, daß** X eine Gruppe Phenyl oder -NHCO-Benzyl darstellt.

6. Verbindung nach einem der Ansprüche 1 bis 3, **dadurch gekennzeichnet, daß** X die Gruppe 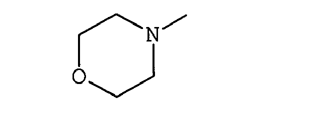 ist.

7. Verbindung nach einem der Ansprüche 1 bis 6, **dadurch gekennzeichnet, daß** R₈ ein Wasserstoffatom ist.

8. Verbindung nach einem der Ansprüche 1 bis 7, **dadurch gekennzeichnet, daß** R₇ ein Wasserstoffatom ist.

9. Verbindung nach einem der Ansprüche 1 bis 8, **dadurch gekennzeichnet, daß** R₆ ein Wasserstoffatom ist.

10. Verbindung nach einem der Ansprüche 1 bis 8, **dadurch gekennzeichnet, daß** R₆ die Gruppe Phenyl, -C₆H₄-O-C₆H₅ oder -C₂H₄-O-C₆H₄Br ist.

11. Verbindung nach einem der Ansprüche 1 bis 10, **dadurch gekennzeichnet, daß** m gleich 0 oder 2 ist.

12. Verbindung nach Anspruch 1, ausgewählt unter den folgenden Formeln:
(3S)-2-{3-[(Phenylacetyl)-amino]-1-oxopropyl}-N-[cyano-(3-phenoxyphenyl)-methyl]-hexahydro-3-pyridazincarboxamid; und
(3S)-N-[Cyano-(3-phenoxyphenyl)-methyl]-2-(1-oxo-3-phenylpropyl)-hexahydro-3-pyridazincarboxamid.

13. Verbindung nach irgendeinem der Ansprüche 1 bis 12, welche die folgende Stereochemie aufweist:

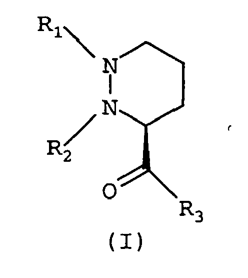

14. Verbindung nach einem der Ansprüche 1 bis 13 für ihre Verwendung als Arzneimittel.

15. Verbindung nach Anspruch 14 für ihre Verwendung als Arzneimittel, das vorgesehen ist zur Vorbeugung oder Behandlung von Erkrankungen, bei denen metabolische Enzyme, ausgewählt unter den Proteasen und den Kinasen, einbezogen sind.

16. Verbindung nach Anspruch 14 oder 15 für ihre Verwendung als Arzneimittel, das vorgesehen ist zur Vorbeugung oder Behandlung von Erkrankungen, bei denen das Cathepsin K einbezogen ist.

17. Verbindung nach Anspruch 15 oder 16, wobei die Erkrankungen, denen vorzubeugen ist oder die zu behandeln sind, aus der Gruppe gewählt werden, die gebildet wird durch cardiovasculäre Erkrankungen, Krebs, Erkrankungen des Zentralnervensystems, inflammatorische Erkrankungen, infektiöse Erkrankungen und Erkrankungen des Knochens.

18. Verbindung nach Anspruch 15 oder 16, wobei die Erkrankungen, denen vorzubeugen ist oder die zu behandeln sind, Osteoporose, Hypercalcämie, Osteopenie, Zahnfleischerkrankungen, Arthritis, Paget-Krankheit, Knochenkrebs sind.

19. Pharmazeutische Zusammensetzung, enthaltend als Wirkstoff mindestens eine Verbindung nach einem der Ansprüche 1 bis 18 in Assoziation mit einem pharmazeutisch annehmbaren Trägerstoff.

20. Verwendung einer Verbindung nach einem der Ansprüche 1 bis 18 für die Herstellung eines Arzneimittels, das vorgesehen ist zur Vorbeugung oder Behandlung von Erkrankungen, bei denen metabolische Enzyme, ausgewählt unter den Proteasen und den Kinasen, einbezogen sind.

21. Verfahren zur Herstellung eines Produktes nach einem der Ansprüche 1 bis 13, **dadurch gekennzeichnet, daß** es die Stufe der Reaktion eines Produktes der Formel (IV) 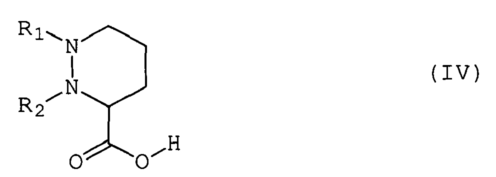 in der R₁ und R₂ die gleiche Bedeutung wie in einem der Ansprüche 1 bis 13 besitzen, mit einem Aminonitril oder Cyanhydrin der Formel HR₃ umfaßt, worin R₃ die gleiche Bedeutung wie in einem der Ansprüche 1 bis 13 besitzt, um ein Produkt der Formel (I) zu erhalten.

22. Verfahren zur Herstellung eines Produktes nach einem der Ansprüche 1 bis 13, **dadurch gekennzeichnet, daß** es umfaßt:
1) eine Stufe, in deren Verlauf man ein Produkt der Formel (II) 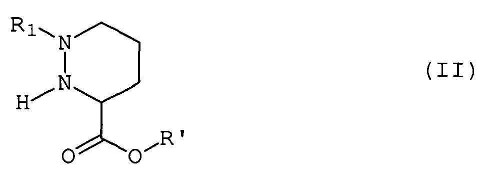 mit einem Säurechlorid der Formel R₂Cl zur Reaktion bringt, worin R₁ und R₂ die gleichen Bedeutungen wie in einem der Ansprüche 1 bis 13 besitzen und R' eine lineare oder verzweigte Gruppe Alkyl mit 1 bis 6 Kohlenstoffatomen ist, um das Produkt der Formel (III) 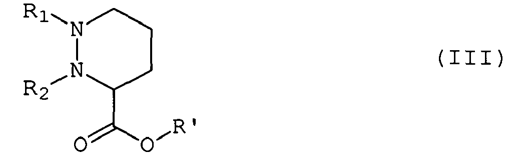 zu erhalten;
2) eine Stufe, in deren Verlauf man das in der Stufe 1) erhaltene Produkt der Formel (III) zu dem Produkt der Formel (IV) 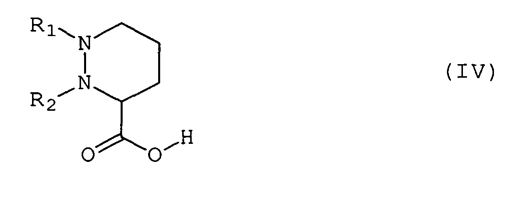 hydrolysiert;
3) eine Stufe, in deren Verlauf man das in der Stufe 2) erhaltene Produkt der Formel (IV) mit einem Aminonitril oder Cyanhydrin der Formel HR₃ zur Reaktion bringt, worin R₃ die gleiche Bedeutung wie in einem der Ansprüche 1 bis 13 besitzt, um ein Produkt der Formel (I) zu erhalten.

23. Verfahren nach Anspruch 21 oder 22, **dadurch gekennzeichnet, daß** es eine Stufe der Hydrogenolyse des Endproduktes umfaßt.

24. Verfahren nach einem der Ansprüche 21 bis 23, **dadurch gekennzeichnet, daß** es eine oder mehrere der folgenden optionalen Reaktionen in einer geeigneten Reihenfolge umfaßt:
- Schutz der reaktiven Funktionen,
- Entschützen der reaktiven Funktionen,
- Veresterung,
- Verseifung,
- Amidierung,
- Acylierung,
- Sulfonylierung,
- Alkylierung,
- Einführung einer Doppelbindung,
- Bildung einer Harnstoffgruppe,
- Reduktion von Carbonsäuren,
- Dehydratisierung des Amids zum Nitril,
- Salzbildung,
- Ionenaustausch,
- Aufspaltung oder Trennung der Diastereoisomeren.
